# EUROPEAN PATENT SPECIFICATION

(11) **EP 3 345 649 B1**
(45) Date of publication and mention of the grant of the patent: **10.08.2022**
(21) Application number: 17799571.9
(22) Date of filing: 14.04.2017
(51) Int. Cl.: A61M 37/00, H01J 37/32

(54) **METHOD FOR MANUFACTURING MICRONEEDLE**
VERFAHREN ZUR HERSTELLUNG VON MIKRONADELN
PROCÉDÉ DE FABRICATION DE MICRO-AIGUILLE

(30) Priority: 20.05.2016 KR 20160061903
(43) Date of publication of application: 11.07.2018
(73) Proprietor: Raphas Co., Ltd., Seoul 03920 (KR)
(72) Inventor: KIM, Jung Dong, Seoul 08364 (KR); JEONG, Do Hyeon, Seoul 03904 (KR); KIM, Beom Joon, Tokyo 1710033 (JP); KIM, Hong Kee, Gunpo-si Gyeonggi-do 15815 (KR)
(74) Representative: Ter Meer Steinmeister & Partner
(86) International application number: PCT/KR2017/004058
(87) International publication number: WO 2017/200213

(56) References cited:
- CN-A- 105 169 552
- KR-A- 20070 042 012
- KR-A- 20110 007 734
- KR-A- 20110 010 665
- KR-A- 20110 110 665
- US-A1- 2008 157 421
- US-A1- 2008 299 290
- US-A1- 2011 240 201

## Description

### Technical Field

The present invention relates to a method of manufacturing a microneedle, and more particularly, to a method of manufacturing a microneedle which is inserted into skin for supplying drugs or nutrients in the body.

### Background Art

Although a great number of drugs and bioactive substances for treating diseases has been developed, the drugs and bioactive substances are hardly delivered into the body due to a biological barrier, for example, skin, oral mucosa, and brain-blood barrier and thus, problems regarding passing through the biological barrier and delivery of drugs still remain.

In general, drugs and bioactive substances are orally administered in the form of tablets or capsules. However, various drugs are digested or absorbed in the gastro-intestinal tract or lost due to a liver mechanism and accordingly, the drugs and bioactive substances may not be efficiently delivered. In addition to, several drugs pass through intestinal mucous membrane and may not be effectively spread. Patient's compliance is also a matter (for example, if a patient needs to take medicine in a regular interval or critical patents who may not take medicine).

Other general way of delivering drugs and bioactive substances is a use of needles. Such a way is more efficient than oral administration, however, may cause pain on injection sites, a local damage on skin, bleeding, and disease infection on injection sites.

In order to solve problems of oral administration and subcutaneous injection, transdermal administration using patches may be used. In the transdermal administration using patches, patient compliance is high and drug level in blood remains steady. However, skin-permeable drugs are limited and drug delivery rate is low.

Accordingly, various micro structures including microneedles are developed. The microneedles developed until the present are mainly used in delivering drugs in the body, blood-gathering, and detecting analyte in the body.

On the one hand side, methods for manufacturing needles are known from prior art documents US 2008/0157421 A1 and US 2011/0240201 A1.

In this context, US 2008/0157421 A1 discloses a skin needle manufacturing apparatus which includes a base on which a needle is erected and a syringe. The syringe can hold and release portions of molten material. Furthermore, the syringe is movable in a direction perpendicular to the base. In this way, a portion of molten material which already adheres to the base but is not completely released from the syringe may be tapered by the tension forces that are generated through an upward movement of the syringe.

US 2011/0240201 A1 describes different processes for manufacturing a microstructure. In all of the described processes at least one viscous composition containing at least one active ingredient is disposed on a first and/or a second substrate and the viscous composition is elongated between the first and second substrates.

On the other hand, a completely new method of manufacturing a micro structure is disclosed in Korean Patent Application No. 10-2010-0130169 (title of the invention: Me thod of manufacturing a micro structure, hereinafter, referred to as a prior art) by the same applicant. In the prior art, a viscous material is spotted in drops on a substrate by using a nozzle and then, the viscous material contacts with another substrate or a viscous material spotted on the other substrate. Then, the contacted viscous material is elongated and coagulated. In addition, according to such method, sufficient hardness may be realized and a loss of functional materials may be reduced.

However, since the viscous material is spotted in drops on a substrate in the prior art, the time required for spotting a number of viscous materials on a substrate increases and thereby, the entire process time increases. Therefore, productivity is reduced.

Also, when the spotting time gets longer, a part of the viscous material that is already spotted is evaporated. Thus, a difference in viscosity between the viscous materials may occur and thereby, a deviation in a length of microneedles or a thickness of the top of the microneedles gets worse. Therefore, the defect rate of products may be increased. In particular, when a thickness of the top of the microneedle increases, efficiency of skin penetration lowers and thereby, drug delivery rate lowers. Also, when a thickness of the top of the microneedle increases, patients' will have severe pain and thus people using the products may feel uncomfortable.

In addition, according to the method of manufacturing a micro structure in the prior art, a force to combine the surface of the substrate and the viscous material together may be needed for elongating and separating and thus, usable viscous materials may be limited according to a characteristic of the surface of the substrate.

### Disclosure

### Technical Problem

The present invention provides a method of manufacturing a microneedle by which the time required to spot a viscous material is reduced and thereby, quality of products and production efficiency may be increased.

### Technical solution

In order to solve the technical problem, the present disclosure provides a method of manufacturing a microneedle according to the features of the independent claim. Preferred embodiments are covered by the dependent claims. The method of manufacturing a microneedle, inter alia, comprises spotting a viscous material on a plurality of points on a film using an injection plate, elongating the viscous material, and coagulating the elongated viscous material.

### Advantageous Effects of Invention

The present disclosure provides a method for manufacturing a microneedle enabling product quality and efficiency in production to be improved by reducing the time needed for spotting a viscous material. The invention is defined in claims 1-6.

### Description of Drawings

The above and other features and advantages of the present invention will become more apparent by describing in detail exemplary embodiments thereof with reference to the attached drawings in which:
FIG. 1 is a flowchart schematically illustrating a method of manufacturing a microneedle according to an embodiment of the present invention;
FIG. 2 is a cross-sectional diagram schematically illustrating a viscous material supply device for manufacturing a microneedle according to an embodiment of the present invention;
FIGS. 3 and 4 illustrate a process of spotting a viscous material by using the viscous material supply device for manufacturing a microneedle of FIG. 2;
FIGS. 5 and 6 are cross-sectional diagrams schematically illustrating viscous material supply devices for manufacturing a microneedle according to other embodiments of the present invention;
FIG. 7 is a cross-sectional diagram schematically illustrating a viscous material supply device for manufacturing a microneedle according to another embodiment of the present invention;
FIG. 8 illustrates a process of spotting a viscous material by using the viscous material supply device for manufacturing a microneedle of FIG. 7;
FIG. 9 illustrates a process of supplying a viscous material by using a viscous material supply device for manufacturing a microneedle according to another embodiment of the present invention;
FIG. 10 is a cross-sectional diagram of an injection plate according to another embodiment of the present invention; and
FIG. 11 is a flowchart of a method of manufacturing a microneedle according to another embodiment of the present invention.

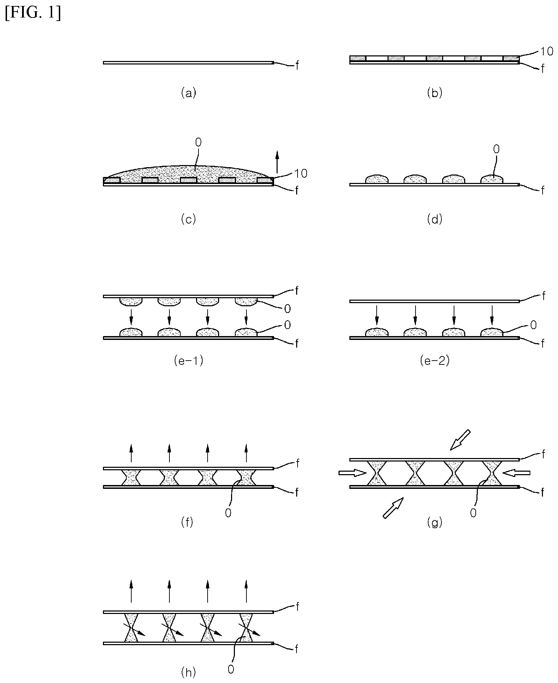

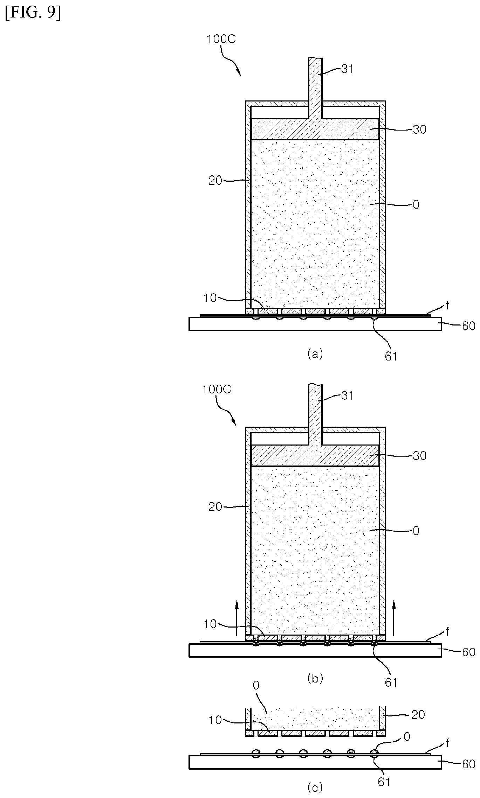

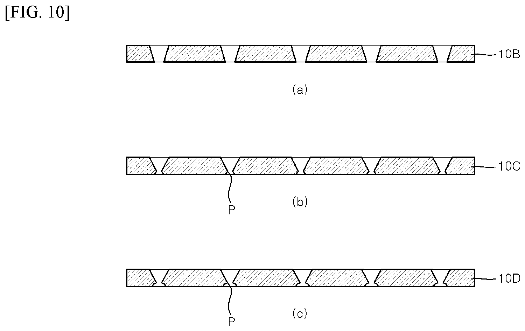

The invention is described in Figs.9 and 10. Other embodiments of manufacturing methods of Figs. 1-8, 11 which do not comprise the filling grooves as in Fig.9 do not form part of the invention and are left for illustrative purposes.

### Best Mode

According to an aspect of the present invention, there is provided a method of manufacturing a microneedle comprising: spotting a viscous material on a plurality of points on a film by supplying the viscous material on the upper surface of the film through a plurality of through holes disposed on an injection plate; elongating the viscous material spotted on the film; and coagulating the elongated viscous material.

The method may further comprise surface modifying the film so as to increase an adhesive force between the viscous material and the film before spotting of the viscous material.

The surface modifying may be performed by plasma treatment.

In the spotting, the viscous material may be supplied while the injection plate is closely adhered to the upper surface of the film.

The injection plate may be surface treated so as to lower an adhesive force between the injection plate and the viscous material.

The through holes of the injection plate may have widths which become narrow from the upper part to the lower part.

According to another aspect of the present invention, there is provided a method of manufacturing a microneedle comprising: disposing a mask, on which a plurality of through holes is formed, on a film; surface modifying the surface of the film that corresponds to the through holes by plasma treating the film so as to increase an adhesive force between the film and the viscous material; contacting the surface modified film with the viscous material and separating the film so as for the viscous material to be adhered to the surface modified part of the film; elongating the viscous material adhered to the film; and coagulating the elongated viscous material.

### Mode for Invention

Hereinafter, a method of manufacturing a microneedle and a viscous material supply device for manufacturing a microneedle according to the exemplary embodiments of the present invention will be described more fully with reference to the accompanying drawings.

FIG. 1 is a flowchart schematically illustrating a method of manufacturing a microneedle according to an embodiment of the present invention.

Firstly, the present invention relates to a method of manufacturing a microneedle by using elongating a viscous material. Here, the viscous material may be "a biocompatible or biodegradable material." The "biocompatible material" denotes a non-toxic and chemically inert material. The "biodegradable material" denotes a material which may be biodegraded by body fluid, enzyme, or microorganism in the body.

In addition, the viscous material may be viscous after being dissolved by a suitable solvent. That is, although the viscous material may have viscosity while being melted by heat, the viscous material according to the present invention may preferably have viscosity while being dissolved by a solvent in order to show a non-heated process, which is one of the best points in the present invention.

The viscous material described above may include a hyaluronic acid and a salt thereof, polyvinyl pyrrolidone, polyvinyl alcohol, cellulose polymer, dextran, gelatin, glycerin, polyethylene glycol, polysorbate, propylene glycol, povidone, carbomer, gum ghatti, guar gum, glucomannan, glucosamine, dammer resin, rennet casein, locust bean gum, microfibrillated cellulose, psyllium seed gum, xanthan gum, arabino galactan, Arabic gum, alginic acid, gellan gum, carrageenan, karaya gum, curdlan, chitosan, chitin, tara gum, tamarind gum, tragacanth gum, furcelleran, pectin, or pullulan. More preferably, the viscous material used in the present invention may be hydroxypropyl methyl cellulose, hydroxyalkyl cellulose, ethyl hydroxyethyl cellulose, alkyl cellulose, carboxymethyl cellulose, and in particular, carboxymethyl cellulose.

In addition, a solvent used to dissolve the viscous material is not particularly restricted and may include water, carbon number of 1-4 of anhydrous or low-level alcohol, acetone, ethyl acetate, chloroform, 1,3-butylene glycol, hexane, diethyl ether, or Butyl Acetate, and more preferably, water or low-level alcohol.

Referring to FIG. 1, the method of manufacturing a microneedle according to an embodiment of the present invention includes spotting, elongating, coagulating, and cutting.

In the spotting, a viscous material is spotted on a plurality of points on a film. In the present invention, it is important that the viscous material is spotted on a plurality of points on a film f once. Here, the film f may be formed of a flexible material so as to be adhered to a skin and may include various forms of film, for example, polyurethane, polyvinyl alcohol, cellulose gum, gelatin, or a film coated by an adhesive composition.

In a basic concept of the spotting, an injection plate 10, on which a plurality of through holes is formed, is placed on the film f as illustrated in (b) of FIG. 1 while the film f is firstly placed in (a) of FIG. 1 and then, a viscous material 0 is supplied to the film f through the through holes of the injection plate 10 ((c) of FIG. 1). Accordingly, the viscous material 0 is spotted on the plurality of points on the film f ((d) of FIG. 1).

Since the spotting is an important feature in the present invention and includes various examples, the spotting will be described in more detail later.

In the elongating, the spotted viscous material 0 is elongated. Here, as illustrated in (e-1) and (f) of FIG. 1, the two films f, on which the viscous material 0 are spotted respectively, face each other, the spotted viscous material 0 contact each other, the two films f are separated from each other, and then the viscous material 0 may be elongated. In addition, as illustrated in (e-2) and (f) of FIG. 1, the film f (or a plate) contacts with the viscous material 0, the film f is separated from the other film f, and thereby, the viscous material 0 may be elongated.

In the coagulating, the elongated viscous material 0 are coagulated. Here, if needed, air may blow as illustrated in (g) of FIG. 1 and thus, coagulating speed and strength of a microneedle may increase.

In the cutting, the two films f are separated from each other rapidly while the viscous material 0 are completely coagulated as illustrated in (h) of FIG. 1. Also, a laser or cutting tools may be used in the cutting.

According to the present invention, modifying of a surface of the film f may be further included before the spotting of the viscous material on the film f. In regard to this, a microneedle is manufactured after spotting of the viscous material on the film and elongating of the viscous material in the present invention. Here, the viscous material may be elongated only when the viscous material are adhered to the surface of the film. Also, an adhesive force between the viscous material and the surface of the film is directly connected to an elongating degree of the viscous material (that is, form of a microneedle). Accordingly, an adhesive force between the viscous material and the surface of the film may be uniform and more preferably, may be over a certain level.

However, types of materials used as a film are substantially limited and a surface of the film is not uniform. Accordingly, in the present invention, a film is plasma processed and thus, a surface is modified. Therefore, an adhesive force between the viscous material and the film increases and the surface of the film is uniform (that is, an adhesive force). Then, the viscous material may be elongated uniformly in the elongating process. Types of plasma and strength may vary according to the type of the film.

According to the present invention, the viscous material may be spotted on a plurality of points on the film at the same time and thus, the spotting time is greatly reduced compared with that of in the prior art. Accordingly, the entire process time is reduced and thus, productivity is increased.

Also, as the spotting time is reduced, problems occurring due to a difference in viscosity between the viscous materials by evaporation and non-uniformity of a microneedle while in elongating may be prevented.

Hereinafter, a device used to spot the viscous material and a process of spotting according to the present invention will be described in detail.

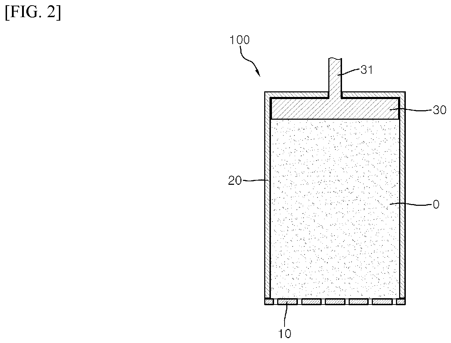

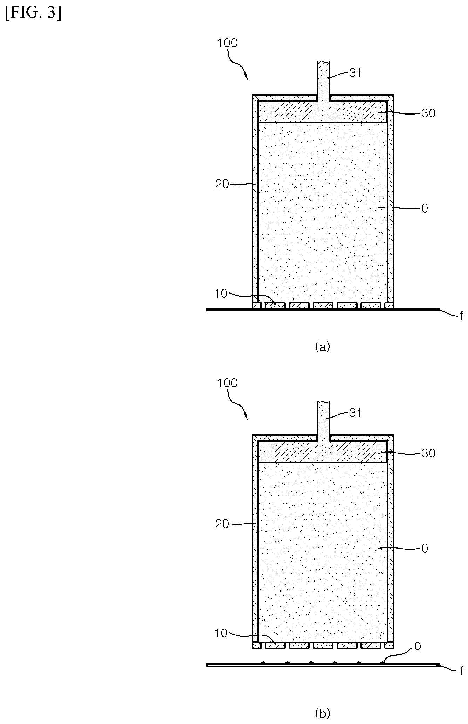

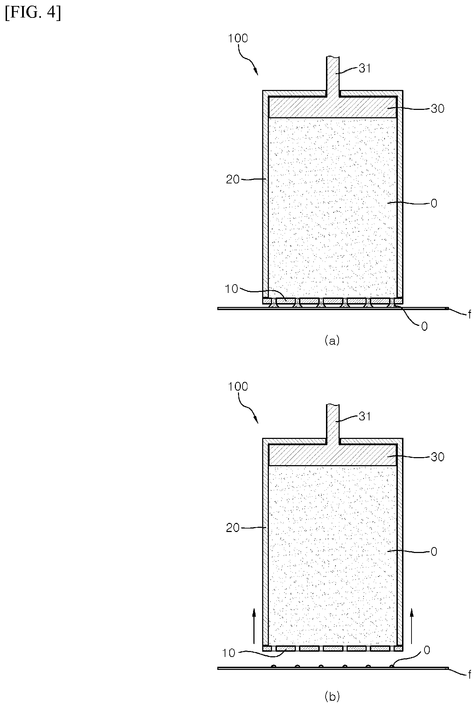

FIG. 2 is a cross-sectional diagram schematically illustrating a viscous material supply device 100 for manufacturing a microneedle according to an embodiment of the present invention and FIGS. 3 and 4 illustrate a process of spotting the viscous material 0 by using the viscous material supply device 100 of FIG. 2 for manufacturing a microneedle.

Referring to FIG. 2, the viscous material supply device 100 for manufacturing a microneedle supplies the viscous material 0 on a plurality of points, which are spaced apart from each other, on the film f. The viscous material supply device 100 for manufacturing a microneedle according to the current embodiment of the present invention includes a main body part 20, an injection plate 10, and a pressure means.

In the main body part 20, a space for storing the viscous material 0 exists and one end of the main body part 20 is opened. The injection plate 10 is formed of a plate and includes a plurality of through holes penetrated therethrough. The injection plate 10 is combined to the one end (the opened part) of the main body part 20. The pressure means applies pressure so that the viscous material 0 stored in the main body part 20 is discharged through the through holes. In the current embodiment of the present invention, the pressure means may be a piston structure. That is, a pressurizing plate 30 is disposed in the main body part 20. When the pressurizing plate 30 moves to the lower part by a drive shaft 31, the viscous material 0 is discharged through the through holes. Also, it is important that a uniform amount of the viscous material 0 is discharged. Accordingly, a form of the pressure means may be changed into various means as mentioned above.

The injection plate 10 may be surface treated so that an adhesive force between the viscous material 0 and the injection plate 10 may be minimized. When the viscous material 0 is strongly adhered to the through holes of the injection plate 10, the viscous material 0 is coagulated while the viscous material 0 is adhered to the through holes and thus the through holes are blocked. Accordingly, there may be a deviation in an amount of discharged viscous material 0. Therefore, the injection plate 20 (in particular, including parts of the through holes) according to the current embodiment of the present invention is coated by using a hydrophobic material, for example, PU and Teflon. For reference, a hydrophobic material is used because a large number of viscous materials is aqueous solution. According to a feature of the viscous material, a surface treatment may be appropriately changed.

In the spotting of the viscous material 0 by using the viscous material supply device 100 for manufacturing a microneedle, the injection plate 10 is completely adhered to the upper surface of the film f, as illustrated in (a) of FIG. 3. Here, the viscous material supply device 100 for manufacturing a microneedle may be pressurized to the lower part so that the injection plate 10 may be adhered to the film for an electromagnetic force (between a stage, on which the film f is placed (not illustrated), and the injection plate 10) may be used to adhere the injection plate 10 to the film f. Here, when the viscous material 0 is pressurized, the viscous material 0 is fully filled with the through holes and a part of the viscous material 0 is adhered to the upper surface of the film f. Then, when the pressure is stopped and the viscous material supply device 100 for manufacturing a microneedle is lifted up, the viscous material 0 is spotted on a plurality of points on the film f, as illustrated in (b) of FIG. 3. As described above, an adhesive force between the film f and the viscous material 0 is treated to be strong and an adhesive force between the injection plate 10 and the viscous material 0 is treated to be weak. Accordingly, the viscous material 0 at above a certain level is adhered to the surface of the film f and remains on the film f. In order to adjust an amount of remaining viscous material 0, a size or shape of the through holes may be appropriately changed.

As illustrated in FIG. 4, while the viscous material supply device 100 for manufacturing a microneedle is spaced apart from the film f, a certain amount of viscous material 0 is discharged and then, the viscous material supply device 100 for manufacturing a microneedle is lifted up. However, in this case, a precise pressure means is needed so that an amount of viscous material 0 discharged through the through holes may be uniform.

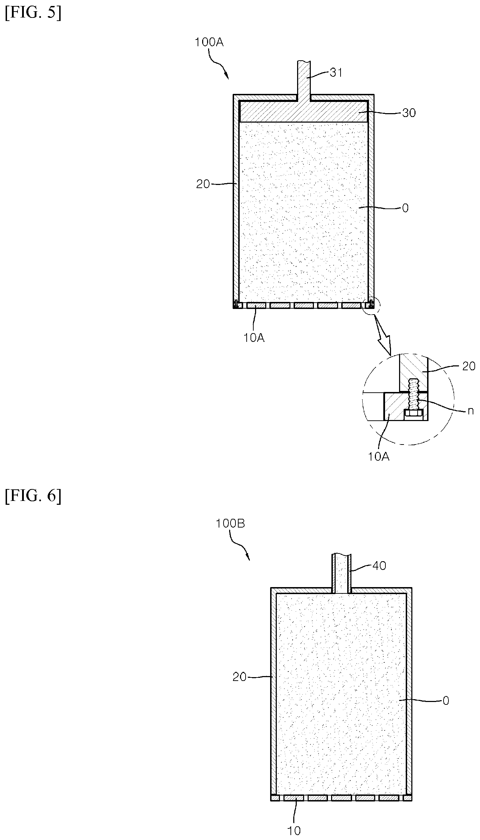

FIGS. 5 and 6 are cross-sectional diagrams schematically illustrating viscous material supply devices 100A and 100B for manufacturing a microneedle according to other embodiments of the present invention.

Referring to FIG. 5, an injection plate 10A is adhered to the main body part 20 by a screw n so as to be detachable. In particular, as illustrated in an enlarged part of FIG. 5, the injection plate 10A includes a groove, in which the screw n is inserted, and the head of the screw n is completely inserted into the groove. Accordingly, the injection plate 10A may be completely adhered to a film.

Referring to FIG. 6, a supply pipe 40 for supplying the viscous material 0 is connected to the main body part 20. Through this supply pipe 40, a certain amount (or certain pressure) of viscous material 0 is supplied and then, the viscous material 0 is discharged through the through holes. For reference, a certain amount of the viscous material 0 may be continuously supplied, however, more preferably, the viscous material 0 may be supplied in a pulse mode. Thus, the viscous material 0 may be supplied at a specific time.

In addition, the viscous material 0 may be supplied on the film by using the viscous material supply devices 100A and 100B for manufacturing a microneedle of FIGS. 5 and 6 as in the same manner as in the viscous material supply device 100 for manufacturing a microneedle of FIGS. 3 and 4.

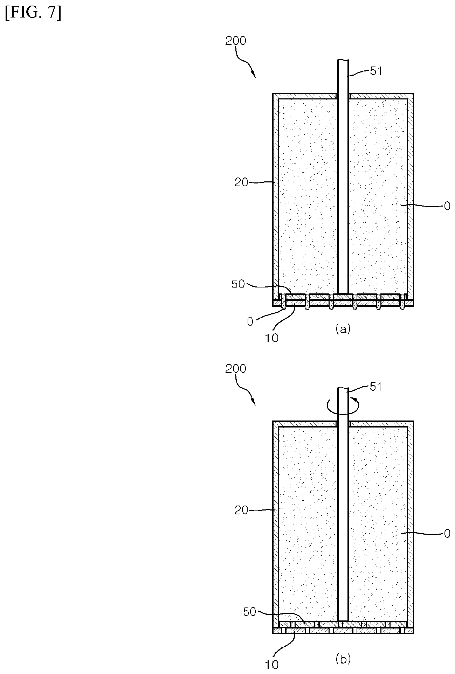

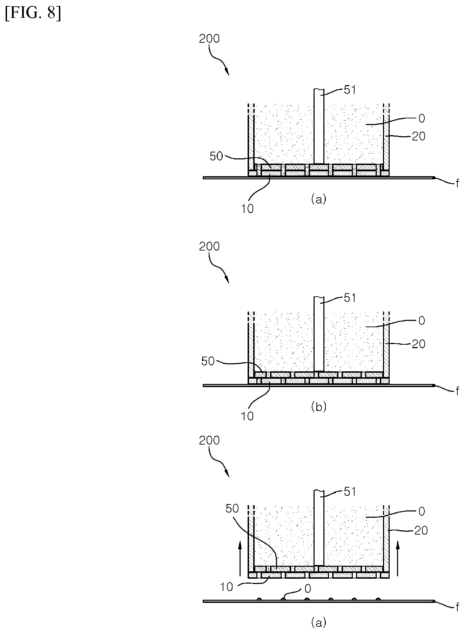

FIG. 7 is a cross-sectional diagram schematically illustrating a viscous material supply device 200 for manufacturing a microneedle according to another embodiment of the present invention and FIG. 8 illustrates a process of spotting the viscous material 0 by using the viscous material supply device 200 for manufacturing a microneedle of FIG. 7.

Referring to FIG. 7, the viscous material supply device 200 for manufacturing a microneedle includes the main body part 20, the injection plate 10, the pressure means, and an opening and closing part 50.

In the main body part 20, a space for storing the viscous material 0 exists and one end of the main body part 20 is opened. The injection plate 10 is formed of a plate and includes a plurality of through holes penetrated therethrough. The injection plate 10 is combined to the one end (the opened part) of the main body part 20. The pressure means applies pressure so that the viscous material 0 stored in the main body part 20 is discharged through the through holes. In the current embodiment, as not illustrated in the drawing, the pressure means may be a supply pipe as in FIG. 6 for supplying the viscous material 0 in the main body part 20.

In addition, the opening and closing part 50 is used to open and close the through holes. In the current embodiment of the present invention, the opening and closing part 50 is formed of a circular plate and is disposed on the upper part of the injection plate 10 inside the main body part 20. The opening and closing part 50 includes a plurality of opening and closing holes penetrated therethrough, each of which corresponds to the through holes of the injection plate 10. The opening and closing part 50 is connected to a rotation axis 51 and moves between an opening position and a closing position as the rotation axis 51 rotates. As illustrated in (a) of FIG. 7, the opening and closing holes and the through holes respectively face each other and accordingly, the opening position is where the viscous material 0 in the main body part 20 may be discharged through the through holes. Also, the closing position is where the through holes are blocked by the opening and closing part 50 since the opening and closing holes and the through holes are disposed alternately as illustrated in (b) of FIG. 7. Here, in the closing position, the viscous material 0 may not be supplied through the through holes.

In the current embodiment of the present invention, the through holes are opened or closed according to rotation of the opening and closing part 50. However, the through holes may be opened or closed according to a sliding of the opening and closing part 50.

Supplying of the viscous material 0 by using the viscous material supply device 200 for manufacturing a microneedle according to the current embodiment of the present invention will be described. Referring to FIG. 8, the injection plate 10 is completely adhered to the film f and the viscous material 0 is supplied through the pressure means while the opening and closing part 50 is disposed in the opening position. Then, as illustrated in (a) of FIG. 8, the viscous material 0 is fully filled with the through holes of the injection plate 10 and thus, a part of the viscous material 0 contacts with the film f. In this state, as illustrated in (b) of FIG. 8, when the opening and closing part 50 rotates toward the closing position, the viscous material 0 is not supplied through the through holes of the injection plate 10.

Then, as illustrated in (c) of FIG. 8, when the injection plate 10 is lifted up, the viscous material 0 existing in the through holes of the injection plate 10 remains and is adhered to the film f and thus, the viscous material 0 may be spotted on a plurality of points. For reference, when an adhesive force between the film f and the viscous material 0 is treated to be strong and when an adhesive force between the injection plate 10 and the viscous material 0 is treated to be weak, the viscous material 0 may be spotted and mostly remain on the film f.

According to the current embodiment of the present invention, an amount of the viscous material spotted at a time is determined according to a volume of the through holes of the injection plate (more preferably, an amount of the supplied viscous material is the same as the volume of the through holes) and accordingly, the viscous material with a constant amount at all times may be spotted. In particular, when the viscous material is injected through the plurality of through holes at the same time as in the current embodiment of the present invention, it is important that an amount of the viscous material injected from each through hole is uniform. An injection amount may be hardly controlled through an injection pressure control (that is, performance of the pressure means) and an advanced skill may be required. However, according to the current embodiment of the present invention, an amount of the injected viscous material may be constant and accurate according to a shape (volume) of the through holes.

FIG. 9 illustrates the process of supplying the viscous material 0 by using a viscous material supply device 100C for manufacturing a microneedle according to the present invention.

Referring to FIG. 9, the viscous material supply device 100C for manufacturing a microneedle according to the present invention further includes a stage 60.

The stage 60 is where the film f is disposed and includes a plurality of filling grooves 61 on the upper surfaces thereof. The filling grooves 61 are concave toward the lower part and are formed to respectively correspond to the through holes of the injection plate 10.

In the supplying of the viscous material 0, the injection plate 10 is completely adhered to the film f while the film f is disposed on the stage 60 as illustrated in (a) of FIG. 9. In this state, when the viscous material 0 is supplied, the film f is pushed and entered the filling grooves 61 due to pressure of supplying the viscous material 0 and the viscous material 0 may be filled with the filling grooves 61 as illustrated in (b) of FIG. 9. Then, when the supplying of the viscous material 0 is stopped and the injection plate 10 is lifted up, the viscous material 0 is spotted as illustrated in (c) of FIG. 9. For reference, as the film f has elasticity at a certain level, the film f may be restored after entering the filling grooves 61. In comparison to the embodiment of FIG. 3, where the viscous material 0 is spotted as much as the volume of the through holes, the viscous material 0 may be spotted more according to the method of the present invention due to the volume of the filling groove 61. Accordingly, an amount of spotted viscous material may be changed by a shape of the filling grooves.

In the process of injecting the viscous material 0 described above, when the lower surface of the injection plate 10 is stained with the viscous material 0 (more specifically, the edge of the through holes) (for example, the viscous material 0 may be leaked outside through the through holes and stained with the lower edge of the through holes), the viscous material 0 is hardened and this will damage the following processes. Accordingly, in the present invention, such a problem is to solve by a change in a shape of the through holes.

FIG. 10 is a cross-sectional diagram of an injection plate 10B according to another embodiment of the present invention.

Referring to (a) of FIG. 10, through holes of the injection plate 10B are formed in a shape of an inverted triangle, in which width thereof becomes narrow from the upper part to the lower part. When the viscous material is pushed to the through holes by applying pressure to the viscous material and the applying pressure is released, the viscous material existing in the through holes is sucked up by its own viscosity. Here, since the width of the lower part of the through holes is narrower than that of the upper part, the viscous material is sucked up much more in the lower part of the through holes, even if the viscous material is sucked up a little in the upper part. Accordingly, the viscous material may be prevented from being leaked outside and stained with the lower surface of the injection plate.

Referring to (b) and (c) of FIG. 10, widths of through holes in injection plates 10C and 10D become narrow from the upper part to the lower part and widens from a specific position p. When the through holes are formed as described above, the viscous material is supplied to a film through the through holes, and then, the injection plates 10C and 10D are lifted up while the supplying of the viscous material is stopped, the viscous material remaining in the through holes is separated by the specific point P. Then, the viscous material existing below the specific position P is adhered to the film f and the viscous material existing above the specific position P remains in the through holes. In addition, when the viscous material leaks through the through holes, the viscous material flows along inner walls of the through holes. In the current embodiment, widths of the inner walls of the through holes widen after the specific point P and thus, the viscous material may be prevented from flowing.

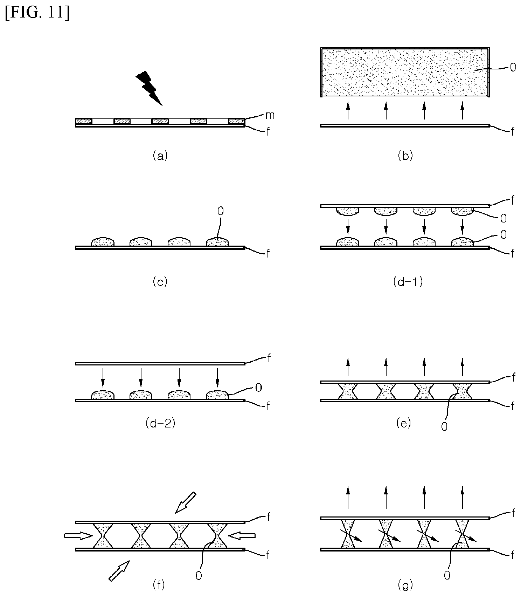

FIG. 11 is a flowchart of a method of manufacturing a microneedle according to another embodiment of the present invention.

Referring to FIG. 11, the method of manufacturing a microneedle according to the current embodiment of the present invention includes spotting, elongating, coagulating, and cutting. The processes except for the spotting are the same as those described with reference to FIG. 1 above. Accordingly, the spotting will be described in detail.

According to the current embodiment of the present invention, as illustrated in (a) of FIG. 11, a mask m, on which a plurality of through holes is formed, is disposed on the film f and plasma treatment is performed. Then, a position exposed to plasma, that is, a surface of the film corresponding to the through holes is modified. Accordingly, an adhesive force between this part (that is, the part that is surface modified) and the viscous material increases.

Then, as illustrated in (b) of FIG. 11, when the film f contacts with the viscous material 0 and then is separated, the viscous material 0 is only adhered to the part having high adhesive force with the viscous material, that is, the part that is surface modified and accordingly, the viscous material 0 is spotted as illustrated in (c) of FIG. 11.

Then, as described above with reference to FIG. 1, elongating, coagulating, and cutting are performed to manufacture a microneedle.

According to the present invention, the viscous material may be easily and simply spotted on the film (without the viscous material supply devices for manufacturing a microneedle described above).

However, when the film contacts the viscous material, the viscous material may be partly stained with the part that is not surface modified). Accordingly, a material having low adhesive force with the viscous material is used to manufacture a film or a part remaining after surface modifying may be surface modified so as to lower an adhesive force with the viscous material.

According to the present invention, the time for spotting the viscous material may be reduced and thus, deterioration due to a viscosity deviation of the deterioration may be prevented. Also, production efficiency may increase.

## Claims

1. A method of manufacturing a microneedle by the use of a viscous material supply device (100C) including a stage (60) and an injection plate (10), wherein the stage (60) has a plurality of filling grooves (61) on its upper surface which are concave toward a lower part and are formed to respectively correspond to through holes of the injection plate (10),
the method comprising the steps of:
spotting a viscous material (0) on a plurality of points on a film (f) by
disposing the film (f) on the stage (60);
supplying the viscous material (0) on the upper surface of the film (f) through the plurality of through holes disposed on the injection plate (10), while the injection plate (10) is completely adhered to the film (f) and the film (f) is disposed on the stage (60);
stopping the supply of the viscous material (0) and lifting up the injection plate (10);
elongating the viscous material (0) spotted on the film (f); and
coagulating the elongated viscous material (0).

2. The method of claim 1, further comprising:
surface modifying the film (f) so as to increase an adhesive force between the viscous material (0) and the film (f) before spotting of the viscous material (0).

3. The method of claim 2, wherein the surface modifying is performed by plasma treatment.

4. The method of claim 1, wherein in the spotting, the viscous material (0) is supplied while the injection plate (10) is closely adhered to the upper surface of the film (f).

5. The method of claim 1, wherein the injection plate (10) is surface treated so as to lower an adhesive force between the injection plate (10) and the viscous material (0).

6. The method of claim 1, wherein the through holes of the injection plate (10) have widths which become narrow from the upper part to the lower part.

## Patentansprüche

1. Verfahren zur Herstellung einer Mikronadel unter Verwendung einer Vorrichtung (100C) zum Zuführen eines viskosen Materials, die ein Gestell (60) und eine Injektionsplatte (10) umfasst, wobei das Gestell (60) auf seiner Oberseite mehrere Einfüllrillen (61) aufweist, die in Richtung eines unteren Teils konkav sind und so ausgebildet sind, dass sie jeweils Durchgangslöchern der Injektionsplatte (10) entsprechen,
wobei das Verfahren die folgenden Schritte umfasst:
Aufbringen eines viskosen Materials (0) auf mehrere Punkte auf einer Folie (f) durch:
Anordnen der Folie (f) auf dem Gestell (60);
Zuführen des viskosen Materials (0) zu der Oberseite der Folie (f) durch die mehreren Durchgangslöcher, die auf der Injektionsplatte (10) angeordnet sind, wobei die Injektionsplatte (10) vollständig an der Folie (f) haftet und die Folie (f) auf dem Gestell (60) angeordnet ist; und
Stoppen der Zufuhr des viskosen Materials (0) und Anheben der Injektionsplatte (10);
Auseinanderziehen des viskosen Materials (0), das auf die Folie (f) aufgebracht wurde; und
Koagulieren des auseinandergezogenen viskosen Materials (0).

2. Verfahren nach Anspruch 1, das ferner den folgenden Schritt umfasst:
Ausführen einer Oberflächenmodifikation der Folie (f), um eine Adhäsionskraft zwischen dem viskosen Material (0) und der Folie (f) vor der Platzierung des viskosen Materials (0) zu erhöhen.

3. Verfahren nach Anspruch 2, wobei die Oberflächenmodifikation durch Plasmabehandlung ausgeführt wird.

4. Verfahren nach Anspruch 1, wobei beim Platzieren das viskose Material (0) zugeführt wird, während die Injektionsplatte (10) direkt an der Oberseite der Folie (f) haftet.

5. Verfahren nach Anspruch 1, wobei die Injektionsplatte (10) oberflächenbehandelt ist, um eine Adhäsionskraft zwischen der Injektionsplatte (10) und dem viskosen Material (0) zu verringern.

6. Verfahren nach Anspruch 1, wobei die Durchgangslöcher der Injektionsplatte (10) Breiten haben, die vom oberen Teil zum unteren Teil hin schmaler werden.

## Revendications

1. Procédé de fabrication d'une micro-aiguille par l'utilisation d'un dispositif de fourniture de matière visqueuse (100C) incluant une platine (60) et une plaque d'injection (10), dans lequel la platine (60) a une pluralité de rainures de remplissage (61) sur sa surface supérieure qui sont concaves vers une partie inférieure et sont formées de manière à correspondre respectivement à des trous traversants de la plaque d'injection (10),
le procédé comportant les étapes consistant à :
déposer une matière visqueuse (0) sur une pluralité de points sur un film (f)
en disposant le film (f) sur la platine (60) ;
en fournissant la matière visqueuse (0) sur la surface supérieure du film (f) à travers la pluralité de trous traversants disposés sur la plaque d'injection (10), alors que la plaque d'injection (10) adhère entièrement au film (f) et que le film (f) est disposé sur la platine (60) ;
en arrêtant la fourniture de la matière visqueuse (0) et en levant la plaque d'injection (10) ;
étirer la matière visqueuse (0) déposée sur le film (f) ; et
faire coaguler la matière visqueuse (0) étirée.

2. Procédé selon la revendication 1, comportant en outre l'étape consistant à :
modifier le film (f) en surface de manière à augmenter une force d'adhérence entre la matière visqueuse (0) et le film (f) avant de déposer la matière visqueuse (0).

3. Procédé selon la revendication 2, dans lequel la modification en surface est réalisée par un traitement par plasma.

4. Procédé selon la revendication 1, dans lequel pendant le dépôt, la matière visqueuse (0) est fournie pendant que la plaque d'injection (10) adhère étroitement à la surface supérieure du film (f).

5. Procédé selon la revendication 1, dans lequel la plaque d'injection (10) est traitée en surface de manière à réduire une force d'adhérence entre la plaque d'injection (10) et la matière visqueuse (0).

6. Procédé selon la revendication 1, dans lequel les trous traversants de la plaque d'injection (10) ont des largeurs qui se rétrécissent de la partie supérieure à la partie inférieure.
